(19) Europäisches Patentamt
European Patent Office
Office européen des brevets

(11) Publication number : **0 527 614 A1**

# (12) EUROPEAN PATENT APPLICATION

(21) Application number : **92307251.6**

(22) Date of filing : **07.08.92**

(51) Int. Cl.$^5$ : **C07C 315/06,** C07C 317/24, C12N 9/78, C12N 9/88, C02F 3/00

(30) Priority : **12.08.91 GB 9117379**

(43) Date of publication of application : **17.02.93 Bulletin 93/07**

(84) Designated Contracting States : **DE ES FR GB IT**

(71) Applicant : **IMPERIAL CHEMICAL INDUSTRIES PLC**
**Imperial Chemical House, Millbank**
**London SW1P 3JF (GB)**

(72) Inventor : **Carver, Mark Andrew**
**503 Yarm Road**
**Eaglescliffe TS16 9BE (GB)**
Inventor : **Daley, Stephen Trevor Adrian Kitchener**
**6 Holly Grove, Lindley**
**Huddersfield, West Yorkshire HD3 3NS (GB)**

(74) Representative : **Locke, Timothy John et al**
**ICI Group Patents Services Dept. PO Box 6**
**Shire Park Bessemer Road**
**Welwyn Garden City Herts, AL7 1HD (GB)**

## (54) Purification of oxidatively labile organic compouds by selectively removing cyanide using a cyanide reacting enzyme.

(57) Cyanide is removed from an aqueous solution of a triacylmethane, especially one having substituents providing herbicidal activity, by treatment with a cyanide reacting enzyme such as cyanide hydratase or cyanide oxygenase or cyanidase. The aqueous solution is for example the product of reacting a benzoyl chloride with a 1,3 cyclohexadione in toluene in the presence of a cyanide catalyst, then extracting the resulting triacylmethane into aqueous alkali.

EP 0 527 614 A1

This invention relates to the purification of labile compound(s), particularly compounds sensitive to oxidation, by removal of cyanide from compositions, particularly aqueous solutions, containing them.

Cyanide is used or is a by-product in many organic synthetic reactions. Its toxic properties necessitate its removal from desired products. Effecting this by physical means, e.g. by precipitation of the desired product, is impracticable if the product is insoluble in acid, unless special precautions are taken to handle the HCN gas liberated on acidification. Oxidation of the cyanide with hypochlorite or peroxide can be satisfactory, but only if the desired product is stable to these materials. Although formaldehyde will react readily with cyanide to remove it from solution, this may not be acceptable in presence of chlorine or chloride because a side reaction can lead to appreciable quantities of bis(chloromethyl)ether, a potent carcinogen.

Among industrially important compounds which could advantageously be made by a synthetic route involving cyanide are so-called 'tri-ketone' herbicides. These are compounds based on the triacylmethane group (I):

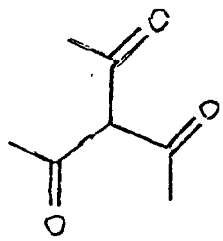

(I)

and typical members include 2-benzoyl-1,3-cyclohexadione compounds having ring structure (II):

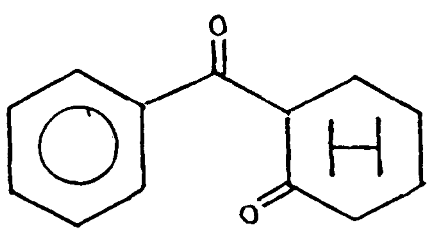

(II)

in which the rings carry various substituents. Compound of this type are described in EP-A-0319 075 and EP-A-0320 864.

Compounds including structure (II) can be made by a route including the following (outline) steps (described as part of a complete synthesis in U.S. 4853028):

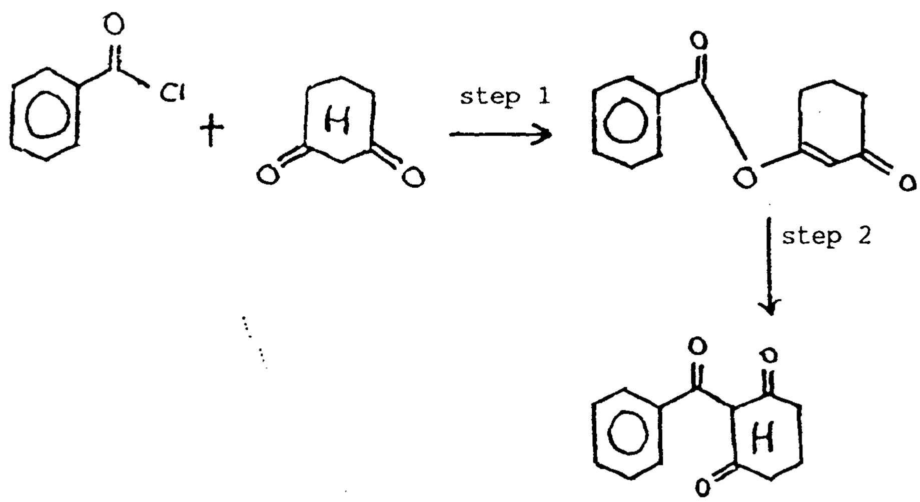


(III)

(II)

Step 1, the formation of enol benzoate, is usually carried out in a hydrocarbon solvent such as toluene in the presence of a base such as triethylamine. The re-arrangement to triacylmethane in step 2 is catalysed by a nucleophile and is typically carried out in the hydrocarbon solvent used in step 1 and often also in the presence

of a phase transfer catalyst. The product is then extracted by addition of dilute aqueous alkali hydroxide. Unfortunately, the only nucleophile known to be efficient in this rearrangement is cyanide. This would be an attractive route to compounds including ring structure (II) were it not for the cyanide present in the reaction mixture. The use of formaldehyde to remove the cyanide is ruled out especially because most practical herbicides including ring structure (II) have halogen, especially chlorine, substituents and because of residual chloride from step 1. The use of oxidising cyanide removers is not practical if the compound including the ring structure (II) is susceptible to oxidation.

Among compounds of value incorporating structure (II) are those described in US 4780127, especially:

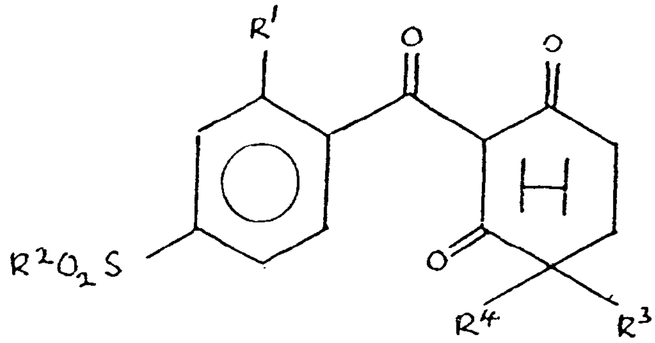

(IIa)

where $R^1$ is halogen, particularly chlorine, or nitro;

$R^2$ is alkyl, especially $C_1$-$C_4$, particularly methyl; and

$R^3$ and $R^4$ are each independently hydrogen or alkyl, especially $C_1$-$C_4$, particularly methyl;

and the aromatic ring optionally carries one or more $C_1$-$C_4$ alkoxy groups

Preferably $R^1$ is Cl, $R^2$ is $CH_3$ or $C_2H_5$ and $R^3$ and $R^4$ are H and $CH_3$ or both H; or $R^1$ is nitro and $R^2 = R^3 = R^4 = CH_3$.

Preferably the optional alkoxy is adjacent to $R^1$.

The invention is based on our finding that cyanide hydratase enzymes are able to effectively and selectively remove cyanide from solutions containing both cyanide and oxidation labile compounds, such as triketone herbicides, without damaging and without themselves being inactivated.

According to the invention a method for selective removal of cyanide in aqueous solution, particularly alkaline aqueous solution, of an oxidatively labile organic compound comprises treating the solution with cyanide reacting enzyme.

The invention in particular provides a method of treating such an aqueous solution of a triacylmethane to selectively remove cyanide by treating it with cyanide hydratase enzyme or enzyme system. This is particularly applicable to triacyl methanes active as herbicides including ring structure (II), especially to compounds of formula (IIa) and those compounds where $R^1$ is chlorine, $R^2$ is methyl and $R^3$ and $R^4$ are each hydrogen or where $R^1$ is nitro and $R^2$, $R^3$ and $R^4$ are each methyl. Further, the invention includes a method of making a triacylmethane including ring structure (II):

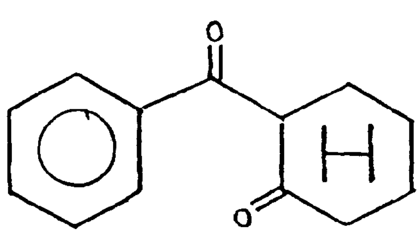

(II)

in which the rings may be substituted as is appropriate to form an effective herbicide by:

(a) re-arranging a compound including corresponding ring structure (III):

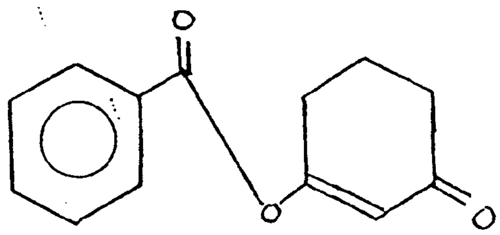

(III)

in presence of a catalytic amount of cyanide;

(b) forming an aqueous solution of the rearrangement product, if necessary adding alkali to the reaction mixture, to give an aqueous solution, particularly an alkaline aqueous solution, of the said triacylmethane and cyanide;

(c) treating the solution by the method of the invention for a time sufficient to substantially reduce the amount of cyanide therein; and

(d) recovering triacylmethane, substantially free from cyanide, from the solution.

This method is particularly applicable to the synthesis of triketone herbicides of formula (IIa) and especially the preferred compounds noted.

By "cyanide reacting enzyme" is meant one capable of promoting a reaction of a cyanide, for example a compound of formula RCN in which R is an alkyl or aryl group or preferably as HCN and/or cyanide ion, in aqueous solution to a less toxic compound. In particular, the reaction is hydrolytic yielding formamide or formate or oxidation yielding nitrogen and an oxidised carbon species such as formaldehyde, formate $CO_2$ or carbonate. Enzymes catalysing the hydrolytic reaction include cyanide hydratase and cyanidase. Cyanide hydratase catalyses the (nominal) reaction:

$$HCN + H_2O \dashrightarrow HCONH_2$$

It is typically used, and is available as, a preparation, typically a whole cell preparation, usually of whole dead cells, of the microorganism that produced it.

Cyanide oxygenase, which catalyses the ( nominal ) reaction:

$$HCN + O_2 + NADH + H^+ \dashrightarrow NH_3 + CO_2 + NAD^+$$

is described in Rollinson G. et al FEMS Microbiology Letters (1987) 40 199-205.

Hydrolytic enzymes, in particular cyanide hydratase, are especially convenient and the invention includes in a specific aspect the use of cyanide hydratase as the cyanide reacting enzyme. Typical source microorganisms include strains of the genus Fusarium, including Fusarium lateritium and in particular the Nees strain CMI 300533 deposited on 3 February 1986, at the Commonwealth Mycological Institute (CMI), Ferry Lane, Kew, Richmond, Surrey, England and published in EP-A-0234760. It is not fully clear whether cyanide hydratase acts on cyanide ion $CN^-$ or hydrogen cyanide HCN or both. It is most effective when both are present, so this is a theoretical rather than a practical problem.

Cyanidase catalyses the (nominal) reaction:

$$HCN + 2H_2O \dashrightarrow HCO_2H + NH_3$$

Like cyanide hydratase, cyanidase is typically used as a preparation, typically a whole cell preparation, of whole dead cells of the microorganism that produced it. A preparation of cyanidase is available from Novo Industries under the trade name "Cyanidase", believed to be a whole cell preparation of Alkaligenes dentrificans strain DSM 30026, NCIB 11962, as described in EP-A-88302186.7 (publication 0282351).

The treatment can be effected by adding the enzyme preparation to the aqueous solution containing cyanide and the oxidatively labile organic compound. It is preferable to add the enzyme preparation in suitably finely divided form e.g. as a dry granules or powder, suitably wetted and/or predispersed in water. The temperature of the treatment will typically be 0 to 50, more usually 20 to 40, and especially about 30°C. At up to about 30°C the activity of the enzyme increases, but at higher temperatures, particularly above about 40°C and especially above about 50°C, the activity falls off. At still higher temperatures it is likely that the enzyme will become denatured. Optimum conditions will depend on the particular enzyme used. The ranges given above are particularly applicable to cyanide hydratase.

The pH of the aqueous medium will typically be in the range 7.5 to 10, especially 8 to 9. At pHs outside this range the activity of the catalyst, particularly cyanide hydratase, is reduced. Further, at lower pHs the cyanide is increasingly present as HCN which can vaporise and if the oxidatively labile organic compound is itself acidic it may precipitate from solution, which is undesirable at this stage. Triketone herbicide compounds as described above are susceptible to such precipitation.

In the catalytic rearrangement, the concentration of cyanide in the solution to be treated will typically be in the range 10 to 10,000 ppm (by weight as $CN^-$), but more usually 200 to 500 ppm. The concentration of enzyme (as whole cell preparation) used will depend on the amount of cyanide to be removed but will usually be in the range 0.05 to 10, more usually 0.1 to 2, $g.l^{-1}$. The time for which the solution is treated will depend on the relative proportions of cyanide and enzyme used and also on other process conditions together with the extent to which it is desired to remove cyanide. Typically, we expect that treatment times in the range 1 to 10, and more usually 3 to 6 h, will be used. Typically, the target final cyanide concentration will be less than 10, usually 1 to 10, ppm. We have satisfactorily achieved levels less than 5 ppm using the present method.

Cyanide reacting enzymes are active in aqueous media and aqueous solution is the most desirable medium for removing cyanide according to the invention. However, the presence of non-aqueous solvents or diluents is not excluded, in particular water immiscible hydrocarbon solvents such as toluene. The method can tolerate such solvents at fairly high concentrations. The presence of large quantities of such solvents can slow down the removal of cyanide, probably because they effectively increase the diffusion barrier for cyanide, thus limiting access to the enzyme. This tolerance to immiscible solvents is important in practice because they are often used in reactions, catalysed by cyanide, which yield oxidatively labile organic compounds (see also below for more detail on triketone herbicide synthesis). Additionally, in such two phase reaction systems, phase transfer catalysts are frequently used and these can partially bind and therefore act as'reservoirs for cyanide present in the system. The present method can satisfactorily remove cyanide from such systems.

After the cyanide removal treatment the enzyme preparation will typically be separated by filtration. This may enable re-use of the enzyme and avoids contaminating the product with the enzyme (if this latter is important). The oxidatively labile organic compound can (then) be recovered. Since many such compounds are relatively insoluble in water under acidic conditions, recovery may simply be accomplished by acidifying the aqueous solution and, if desired, (see below) separating the precipitate.

In its application to the synthesis of triketone herbicides some further explanation may aid understanding of this invention. The condensation reaction in Step 1 is typically the direct reaction, in solution, of a benzoyl chloride with a 1,3-cyclohexanedione in the presence of a base e.g. triethylamine, to remove the HCl produced. The rearrangement in Step 2 can be carried out in a similar, most usually the same, solvent and runs satisfactorily under the same general conditions as Step 1. The source of cyanide is typically an organic compound soluble in the reaction medium and capable of liberating cyanide, such as a ketone cyanohydrin e.g. acetone cyanohydrin. Although the chemical reaction proceeds through these two steps, Steps 1 and 2 can be carried out as a "one pot" process without separation of the O-acyl ester intermediate and with all the reagents present simultaneously. This is convenient because the moisture sensitivity of the benzoyl chloride and the toxic cyanide catalyst each require the use of closed or sealed apparatus. The source of cyanide is conveniently an inorganic salt such as sodium cyanide, an organic salt e.g. quaternary ammonium cyanide, as an alternative to ketone cyanohydrin. To speed up reaction of inorganic cyanide, phase-transfer catalysts such as tetrabutylammonium chloride or 18-crown-6 ether may be added. The product triketone is typically extracted from the organic solution into aqueous alkali, conveniently about IM aqueous alkali hydroxide such as NaOH. The pH at this stage is likely to be greater than 12 and prior to enzyme treatment the mix will be adjusted by adding acid to bring the pH to that desired for the treatment, most usually 8 to 9. Care should be taken to avoid over-acidification even locally, as this may lead to precipitation of the product (as the 'free acid') which will typically re-dissolve only slowly in the modestly alkaline solution.

Further processing of the mix will usually involve separating the enzyme preparation by filtration and subsequent precipitation of the triketone by acidification. Since herbicidal formulations can conveniently use the triketone as an aqueous slurry, it is not necessary to separate all the liquid phase from the precipitated solid triketone. In this aspect, it is a particular advantage of the invention that the precipitated triketone can be used as a slurry in the aqueous phase of the reaction mixture without needing further purification.

The following Examples illustrate the invention. All parts and percentages are by weight unless otherwise indicated.

Analytical methods

HPLC was conducted using a Hypersil H5 ODS column (from Highcrom) using a mixture of water and tetrahydrofuran (4:1 by volume) containing acetic acid (0.5% w/v) and orthophosphoric acid (0.1% w/v). The eluent flow rate was 1 ml. $min^{-1}$ at a temperature of 40°C. The sample size was 10 $\mu l$ and the detector wavelength was 254 nm. Observed retention time of the triketone product was 10.5 min and of the O-acyl ester intermediate 12.0 min.

Cyanide analysis was by an Orion cyanide selective electrode type 9406 fitted to a Orion EA 940 ion analyser standardised against 1, 10 and 100 ppm, cyanide solution standards. A 10 ml aliquot of test sample was diluted with water to 100 ml and the pH adjusted to above 10 (NaOH solution) prior to analysis.

Example 1

Thionyl chloride (4.0g; 33.6 mmol), was added slowly to a stirred suspension of 2-chloro-4-methanesulphonylbenzoic acid (6.1g; 26mmol) in dimethylformamide (DMF) (0.2g; 2.73 mmol) and toluene (45ml) at 70°C under a calcium chloride guard tube. Stirring was continued for a further 2 h at 80°C. The reaction mixture was cooled to 50°C and then transferred to a rotary evaporator and the solvent (mainly toluene) removed under vacuum to give a fawn solid. Further toluene (45ml) was added to the solid product which was then again rotary evaporated to remove any residual thionyl chloride as an azeotrope with the toluene.

Toluene (45ml) was added to the solid residue and the mixture stirred, under a calcium chloride guard tube, until the solid dissolved. This solution was added, using a pasteur pipette, to ensure minimum entry of water, to a stirred solution of 1,3-cyclohexadione (3.31g; 29 mmol), trimethylamine (7.21g; 71.4 mmol) and acetonecyanhydrin (0.085g; 1 mmol) in toluene (20 ml) at 40°C so that the temperature during addition did not exceed 50°C. The reaction mixture was stirred for further 3 h, at which time sample analysis by HpLC showed that the reaction sequence was complete. The reaction mixture was quenched into aqueous NaOH solution (70 ml at 4% w/v) and the phases separated. The cyanide concentration of the aqueous phase was measured as 212 ppm by weight. Phosphoric acid (50% w/v aqueous solution, approx. 1.4 ml) was added to the aqueous phase to adjust the pH to about 8 and cyanide hydratase enzyme preparation based on Fusarium lateritium CMI 300533 ('Cyclear' from ICI, 0.05g) was added and the mixture held at ambient temperature (about 20°C). The cyanide concentration was measured after 1 h (about 39 ppm) and it was noted that the pH had fallen to about 7 and it was readjusted to about 8 by addition of NaOH solution (10% w/v). After 2.5 h of enzyme treatment, the measured cyanide concentration was about 9 ppm. The enzyme preparation was removed by filtration and washed with water (10 ml). The triketone product was isolated by acidification to pH 2.6 using sulphuric acid (50% w/v), filtration and drying on a rotary evaporator at 50°C. The triketone product was obtained as a light yellow solid; yield 7.52g; 23.2 mmol; 89% of theory (allowing for loss in cyanide analysis).

**Claims**

**1** A method for selectively removing cyanide from an aqueous solution of an oxidatively labile organic compound by treating the solution with cyanide reacting enzyme.

**2** A method according to Claim 1 in which the compound is a triacylmethane of formula IIa

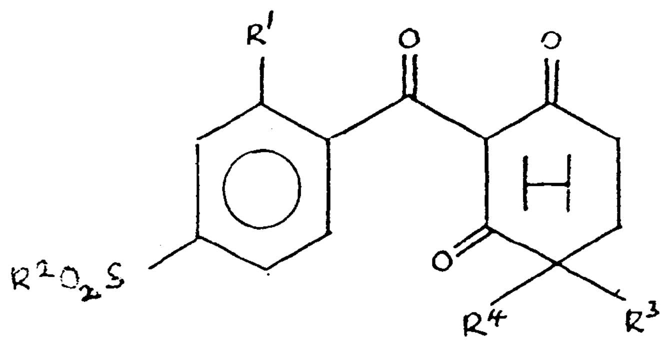

(IIa)

where

$R^1$ is halogen or nitro;

$R^2$ is alkyl; and

$R^3$ and $R^4$ are each independently hydrogen or alkyl; and the aromatic ring optionally carries one or more alkoxy groups.

**3** A method according to Claim 2 in which

$R^1$ is Cl, $R^2$ is $CH_3$ or $C_2H_5$ and $R_3$ and $R_4$ are H and $CH_3$ or both H; or in which

$R^1$ is nitro and $R^2 = R^3 = R^4 = CH_3$.

**4** A method according to any one of the preceding claims in which the enzyme is selected from the group consisting of cyanide hydratase, cyanidase and cyanide oxygenase.

**5** A method according to any one of the preceding claims in which the enzyme is used as a preparation of whole dead cells of the microorganism that produced it.

**6** A method according to Claim 5 in which the enzyme is cyanide hydratase and is used as whole dead cells of Fusarium lateritium.

**7** A method of making a triacylmethane including ring structure II

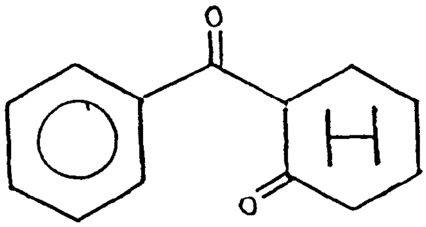

(II)

by the steps of

(a) rearranging a compound including corresponding ring structure (III);

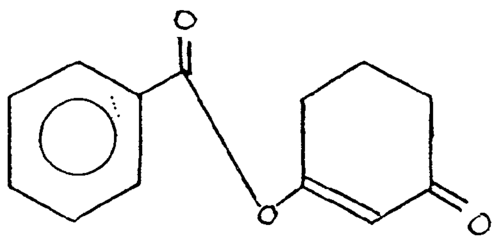

in presence of a catalytic amount of cyanide;

(b) forming an aqueous solution of the rearrangement product, if necessary with addition of alkali;

(c) treating the aqueous solution according to any one of the preceding claims;

(d) recovering substantially cyanide-free triacylmethane from the treated solution.

**8** A method according to Claim 7 in which: step (a) is carried out in a hydrocarbon solvent and the source of cyanide is a ketone cyanhydrin; in step (b) the rearrangement product is extracted from organic solution into aqueous alkali hydroxide; and for step (c) the resulting solution is adjusted by adding acid to pH 8 to 9;

**9** A herbicidal formulation including a triacylmethane as made by the method of any one of the preceding claims.

**10** A method of making a herbicidal formulation by incorporating into it a triacylmethane in the form of the aqueous slurry made by a method according to any one of the preceding claims.

European Patent Office

# EUROPEAN SEARCH REPORT

Application Number
EP 92 30 7251
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EP-A-0 234 760 (IMPERIAL CHEMICAL INDUSTRIES PLC)<br>* page 10; claim 10 *<br>* page 6, line 16 - line 18 *<br>--- | 1-4 | C07C315/06<br>C07C317/24<br>C12N9/78<br>C12N9/88<br>C02F3/00 |
| D,X | EP-A-0 282 351 (NOVO INDUSTRI A/S)<br>* page 11; claims 29-31 *<br>* page 4, line 28 - line 40 *<br>--- | 1-4 | |
| X | EP-A-0 061 249 (IMPERIAL CHEMICAL INDUSTRIES PLC)<br>* page 12; claim 1 *<br>* page 5, line 19 - line 23 *<br>--- | 1-4 | |
| X | CHEMICAL ABSTRACTS, vol. 108, no. 2, 11 January 1988, Columbus, Ohio, US; abstract no. 10678q,<br>P.M. CLARKE 'Enzymatic treatment of cyanide bearing effluents.'<br>page 293 ;column 2 ;<br>* abstract *<br>& IMMOBILISATION IONS BIO-SORPTION 1986, pages 245 - 256<br>--- | 1-4 | TECHNICAL FIELDS SEARCHED (Int. Cl.5 ) |
| X | CHEMICAL ABSTRACTS, vol. 114, no. 14, 8 April 1991, Columbus, Ohio, US; abstract no. 128339g,<br>S. BASHEER ET AL. 'Enzymic degradation of cyanide.'<br>page 365 ;column 1 ;<br>* abstract *<br>& DECHEMA BIOTECHNOL. CONF. 1989, pages 819 - 822<br>---<br>-/-- | 1-4 | C07C<br>C12N |

The present search report has been drawn up for all claims

| Place of search | Date of completion of the search | Examiner |
|---|---|---|
| THE HAGUE | 19 OCTOBER 1992 | FINK D.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number
EP 92 30 7251
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | CHEMICAL ABSTRACTS, vol. 114, no. 21, 27 May 1991, Columbus, Ohio, US; abstract no. 205426n, ANON 'Enzymic method for removal of cyanide and urethane from alcoholic beverages.' page 663 ;column 2 ; * abstract * & RES. DISCL. 1990, page 983 | 1-4 | |
| A | EP-A-0 264 859 (STAUFFER CHEMICAL COMPANY) * page 6, line 55 - page 7, line 29 * * page 9; example 2 * | 1-8 | |

TECHNICAL FIELDS SEARCHED (Int. Cl.5 )

The present search report has been drawn up for all claims

| Place of search | Date of completion of the search | Examiner |
|---|---|---|
| THE HAGUE | 19 OCTOBER 1992 | FINK D.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)